# FASCICULE DE BREVET EUROPEEN

(11) **EP 3 410 443 B1**
(45) Date de publication et mention de la délivrance du brevet: **20.09.2023**
(21) Numéro de dépôt: 18175040.7
(22) Date de dépôt: 30.05.2018
(51) Int. Cl.: G16H 50/50, G16H 50/20, G16H 20/30, G16H 20/40, A61C 7/00

(54) **PROCEDE DE DETERMINATION D'UN TRAITEMENT ORTHODONTIQUE**
VERFAHREN ZUR BESTIMMUNG EINER KIEFERORTHOPÄDISCHEN BEHANDLUNG
METHOD FOR DETERMINING AN ORTHODONTIC TREATMENT

(30) Priorité: 30.05.2017 FR 1754755
(43) Date de publication de la demande: 05.12.2018
(73) Titulaire: Dental Monitoring, 75017 Paris (FR)
(72) Inventeur: SALAH, Philippe, 93170 Bagnolet (FR); PELLISSARD, Thomas, 92110 Clichy (FR); GHYSELINCK, Guillaume, 59169 Cantin (FR); DEBRAUX, Laurent, 75020 Paris (FR)
(74) Mandataire: Cabinet Nony

(56) Documents cités:
- FR-A1- 3 027 505
- US-A1- 2005 026 102
- US-A1- 2016 228 212
- US-A1- 2016 310 235

## Description

### Domaine technique

La présente invention concerne un procédé de détermination d'un instant de basculement à partir duquel un patient portant un appareil orthodontique à arc et attaches portera une ou plusieurs gouttières orthodontiques.

L'invention concerne également un programme informatique pour la mise en oeuvre de ce procédé.

### Etat de la technique

Parmi les appareils orthodontiques, on distingue les appareils orthodontiques à arc et attaches d'une part, et les gouttières orthodontiques d'autre part.

Un appareil orthodontique à arc et attaches comporte des attaches, ou *« brackets »,* fixées sur les dents et reliées entre elles au moyen d'un arc, classiquement en un matériau à mémoire de forme. Il exerce une action rapide sur le déplacement des dents du patient traité. Cependant, cette action décroît progressivement et il est nécessaire que le patient prenne régulièrement rendez-vous chez l'orthodontiste afin de modifier le réglage de l'arc ou le changer. Par ailleurs, un appareil orthodontique à arc et attaches est souvent inesthétique.

Une gouttière, *« aligner »* en anglais, se présente classiquement sous la forme d'un appareil monobloc amovible, classiquement en un matériau polymère transparent. Elle comporte une goulotte conformée pour que plusieurs dents d'une arcade, généralement toutes les dents d'une arcade, puissent y être logées. La forme de la goulotte est adaptée pour maintenir en position la gouttière sur les dents, tout en exerçant une action de correction du positionnement de certaines dents. Une gouttière orthodontique a une action initiale plus lente que celle d'un appareil orthodontique à arc et attaches. Avantageusement, la gouttière peut être cependant remplacée par le patient lui-même. En outre, les gouttières sont plus discrètes que les appareils à arc et attaches.

Un traitement orthodontique « mixte » permet de bénéficier des avantages de chacun de ces deux types d'appareil orthodontique. Il comporte en effet classiquement une première phase de traitement pendant laquelle le patient porte un appareil orthodontique, ou « phase à arc », suivie, à partir d'un instant de basculement, d'une deuxième phase de traitement au moyen de gouttières orthodontiques, ou « phase à gouttières ». Le traitement se poursuit ensuite avec des gouttières.

L'état de la technique le plus proche est décrit dans US 2005/026102 A1, FR 3 027 505 A1, US 2016/310235 A1 et US 2016/228212 A1. Il existe un besoin permanent pour un procédé permettant d'optimiser un traitement mixte, en particulier pour en réduire la durée.

Un but de l'invention est de répondre à ce besoin.

### Résumé de l'invention

L'invention fournit un procédé de détermination, par la mise en oeuvre d'un programme d'ordinateur, d'un instant de basculement optimal d'un traitement orthodontique mixte de dents d'un patient, ledit procédé comportant les opérations suivantes :
1) de préférence en début de traitement ou avant le début du traitement, réalisation d'un modèle tridimensionnel numérique d'au moins une partie d'une arcade portant lesdites dents, ou « modèle de référence initial », et pour chacune desdites dents, définition d'un modèle de référence tridimensionnel numérique de ladite dent dans le modèle de référence initial, ou « modèle de dent » ;
2) après l'opération 1), de préférence en début de traitement ou avant le début du traitement, déformation du modèle de référence initial, par déplacement des modèles de dent, jusqu'à ce que lesdits modèles de dents soient dans un positionnement objectif, de manière à obtenir un « modèle de référence objectif »,
3) après l'opération 2), à un instant d'une phase du traitement pendant laquelle le patient porte un appareil orthodontique à arc et attaches, dit « instant actuel », acquisition, au moyen d'un appareil d'acquisition d'images, d'au moins une image bidimensionnelle desdites dents, dite « image actualisée », dans des conditions d'acquisition réelles ;
4) déformation du modèle de référence initial, par déplacement des modèles de dent, jusqu'à ce que ladite au moins une image actualisée corresponde à une vue du modèle de référence initial ainsi déformé, dit « modèle de référence actualisé », la recherche du modèle de référence actualisé étant de préférence effectuée au moyen d'une méthode métaheuristique, de préférence évolutionniste, de préférence par recuit simulé ;
5) détermination, à partir dudit modèle de référence actualisé et dudit modèle de référence objectif, d'une pluralité de scénarios de traitement résiduel pour déplacer lesdites dents depuis leur position représentée dans le modèle de référence actualisé jusqu'à leur position représentée dans le modèle de référence objectif, chaque scénario comportant, à partir d'un instant de basculement, au moins une phase de traitement au moyen d'une gouttière orthodontique ;
6) détermination, pour chaque scénario, d'un profil comportant au moins une valeur pour un paramètre d'évaluation choisi dans le groupe formé par :
   - la durée du traitement résiduel dudit scénario ;
   - la durée de traitement complet ;
   - un coefficient de complexité clinique ou « score de sévérité » ;
   - un coefficient de douleur pour le traitement résiduel dudit scénario,
   - un coefficient de douleur pour le traitement complet;
   - la durée de la ou des phases de traitement au moyen d'un arc orthodontique à arc et attaches pendant le traitement résiduel ;
   - un coefficient de confort pour le traitement résiduel dudit scénario,
   - un coefficient de confort pour le traitement complet ;
   - un coût pour le traitement résiduel dudit scénario,
   - un coût pour le traitement complet,
   - une fonction des paramètres d'évaluation précédents ;
7) évaluation de chaque profil au moyen d'une règle d'évaluation; et
8) détermination, à partir du scénario dont le profil est optimal, ou « scénario optimal », d'un instant de basculement optimal pour remplacer l'appareil orthodontique à arc et attaches par une gouttière.

Comme on le verra plus en détail dans la suite de la description, un procédé selon l'invention permet d'adapter le traitement à des contraintes spécifiques, et en particulier à des contraintes imposées par le patient. Par exemple, si le patient impose une durée maximale de traitement de six mois et une phase à gouttières la plus longue possible, le procédé selon l'invention permet de tester différents scénarios afin de rechercher le profil répondant le mieux à ce besoin.

Les opérations 5) à 8) peuvent être successives ou non. Quand elles sont successives, elles sont appelées « étapes » 5') à 8'), respectivement.

Dans un mode de réalisation préféré, elles sont imbriquées et constituent les étapes 5") à 8") successives suivantes :
5") détermination, à partir dudit modèle de référence actualisé et dudit modèle de référence objectif, d'un scénario de traitement résiduel pour déplacer lesdites dents depuis leur position représentée dans le modèle de référence actualisé jusqu'à leur position représentée dans le modèle de référence objectif, un scénario de traitement résiduel comportant, à partir d'un instant de basculement, au moins une phase de traitement au moyen d'une gouttière orthodontique ;
6") détermination, pour ledit scénario, d'un profil comportant au moins une valeur pour un paramètre d'évaluation choisi dans le groupe formé par :
   - la durée du traitement résiduel dudit scénario ;
   - la durée de traitement complet ;
   - un coefficient de complexité clinique ou « score de sévérité » ;
   - un coefficient de douleur pour le traitement résiduel dudit scénario ;
   - un coefficient de complexité clinique ;
   - un coefficient de douleur pour le traitement complet ;
   - la durée de la ou des phases de traitement au moyen d'un arc orthodontique à arc et attaches pendant le traitement résiduel ;
   - un coefficient de confort pour le traitement résiduel dudit scénario,
   - un coefficient de confort pour le traitement complet ;
   - un coût pour le traitement résiduel dudit scénario,
   - un coût pour le traitement complet,
   - une fonction des paramètres d'évaluation précédents ;
7") évaluation du profil au moyen d'une règle d'évaluation et, si le profil n'est pas optimal au regard de la règle d'évaluation, détermination d'un nouveau scénario de traitement résiduel et reprise à l'étape 6") ;
8") détermination, à partir du scénario dont le profil est optimal, ou « scénario optimal », d'un instant de basculement optimal pour remplacer l'appareil orthodontique à arc et attaches par une gouttière.

De préférence, un procédé selon l'invention présente encore une ou plusieurs des caractéristiques optionnelles suivantes :
- plus de 5, 10, 50 ou 100 scénarios sont déterminés à l'opération 5) ;
- à l'opération 3), on prend l'image actualisée au moyen d'un appareil d'acquisition d'images choisi dans le groupe constitué par un téléphone mobile, un appareil photo connecté, une montre intelligente, une tablette et ou un ordinateur personnel, fixe ou portable ;
- l'opération 4) comporte les étapes suivantes :
   c) analyse de l'image actualisée et réalisation d'une carte actualisée relative à une information discriminante ;
   d) optionnellement, détermination, pour l'image actualisée, de conditions d'acquisition virtuelles grossières approximant les conditions d'acquisition réelles de ladite image actualisée;
   e) recherche, pour l'image actualisée, d'un modèle de référence actualisé correspondant au positionnement des dents lors de l'acquisition de l'image actualisée, la recherche étant de préférence effectuée au moyen d'une méthode métaheuristique, de préférence évolutionniste, de préférence par recuit simulé ;
- l'étape e) comporte les étapes suivantes :
   e1) définition d'un modèle de référence à tester comme étant le modèle de référence initial puis,
   e2) suivant les étapes suivantes, test de conditions d'acquisition virtuelles avec le modèle de référence à tester afin d'approximer finement lesdites conditions d'acquisition réelles ;
      e21) détermination de conditions d'acquisition virtuelles à tester;
      e22) réalisation d'une image de référence bidimensionnelle du modèle de référence à tester dans lesdites conditions d'acquisition virtuelles à tester ;
      e23) traitement de l'image de référence pour réaliser au moins une carte de référence représentant, au moins partiellement, ladite information discriminante ;
      e24) comparaison des cartes actualisée et de référence de manière à déterminer une valeur pour une première fonction d'évaluation, ladite valeur pour la première fonction d'évaluation dépendant des différences entre lesdites cartes actualisée et de référence et correspondant à une décision de poursuivre ou de stopper la recherche de conditions d'acquisition virtuelles approximant lesdites conditions d'acquisition réelles avec plus d'exactitude que lesdites conditions d'acquisition virtuelles à tester déterminées à la dernière occurrence de l'étape e21) ;
      e25) si ladite valeur pour la première fonction d'évaluation correspond à une décision de poursuivre ladite recherche, modification des conditions d'acquisition virtuelles à tester, puis reprise à l'étape e22) ;
   e3) détermination d'une valeur pour une deuxième fonction d'évaluation, ladite valeur pour la deuxième fonction d'évaluation dépendant des différences entre les cartes actualisée et de référence dans les conditions d'acquisition virtuelles approximant au mieux lesdites conditions d'acquisition réelles et résultant de la dernière occurrence de l'étape e2), ladite valeur pour la deuxième fonction d'évaluation correspondant à une décision de poursuivre ou de stopper la recherche d'un modèle de référence approximant le positionnement des dents lors de l'acquisition de l'image actualisée avec plus d'exactitude que ledit modèle de référence à tester utilisé à la dernière occurrence de l'étape e2), et si ladite valeur pour la deuxième fonction d'évaluation correspond à une décision de poursuivre ladite recherche, modification du modèle de référence à tester par déplacement d'un ou plusieurs modèles de dents, puis reprise à l'étape e2) ;
- le procédé comporte une étape 9) dans laquelle l'instant de basculement optimal, de préférence le scénario optimal et/ou le profil optimal, sont présentés à un docteur, en particulier un orthodontiste, et/ou au patient ;
- à l'étape 1), on définit un modèle de référence tridimensionnel numérique d'une gencive de laquelle émergent lesdits modèles de dents, ou « modèle de gencive », et, à l'étape 4), on déplace lesdits modèles de dent et on modifie ledit modèle de gencive pour déformer le modèle de référence initial ;
- pour déformer le modèle de référence initial, on calcule une déformation du modèle de gencive à partir desdits déplacements desdits modèles de dent,
   - de préférence les opérations 4) et/ou 5) et/ou 6) et/ou 7) et/ou 8), de préférence au moins les étapes 4) et 5) et de préférence 6), et de préférence 7) et de préférence 8) sont réalisées avec un ordinateur ;
   - un orthodontiste commande de préférence l'ordinateur à l'étape 2) ;

L'invention concerne aussi un procédé d'adaptation d'une gouttière orthodontique, procédé dans lequel on met en oeuvre un procédé de détermination d'un instant de basculement optimal selon l'invention, puis, en fonction du résultat de ladite évaluation, on fabrique une gouttière adaptée pour au moins une partie du traitement orthodontique des dents à partir dudit instant de basculement optimal.

L'invention concerne également :
- un programme d'ordinateur, et en particulier un applicatif spécialisé pour téléphone mobile, comprenant des instructions de code de programme pour l'exécution d'une ou plusieurs, de préférence toutes les opérations 3) à 8), lorsque ledit programme est exécuté par un ordinateur,
- un support informatique sur lequel est enregistré un tel programme, par exemple une mémoire ou un CD-ROM, et
- un outil de calcul, en particulier un appareil personnel, en particulier un téléphone mobile ou une tablette, dans lequel est chargé un tel programme.

De préférence, l'outil de calcul comporte un simulateur de scénarios apte à créer des scénarios, à déterminer des profils desdits scénarios et à évaluer lesdits scénarios au regard d'une règle d'évaluation. De préférence encore, il comporte une interface « homme-machine » et des moyens pour déterminer la règle d'évaluation à partir de données saisies avec ladite interface.

L'invention concerne aussi un système comportant
- un scanner tridimensionnel apte à réaliser un modèle de référence initial, et
- un outil de calcul, en particulier un appareil personnel, de préférence un téléphone mobile, apte à acquérir une image actualisée et chargé avec un programme selon l'invention.

### Définitions

Par « traitement résiduel », on entend la partie du traitement à partir de l'instant actuel.

Par « patient », on entend toute personne pour laquelle un procédé selon l'invention est mis en oeuvre, que cette personne soit malade ou non.

Les « conditions d'acquisition » précisent la position et l'orientation dans l'espace d'un appareil d'acquisition d'images relativement aux dents du patient (conditions d'acquisition réelles) ou à un modèle tridimensionnel des dents du patient (conditions d'acquisition virtuelles), et de préférence la calibration de cet appareil d'acquisition d'images. Des conditions d'acquisition sont dites "virtuelles", ou « simulées », lorsqu'elles correspondent à une simulation dans laquelle l'appareil d'acquisition serait dans lesdites conditions d'acquisition (positionnement et de préférence calibration virtuelles de l'appareil d'acquisition).

La « calibration » d'un appareil d'acquisition est constituée par l'ensemble des valeurs des paramètres de calibration. Un "paramètre de calibration" est un paramètre intrinsèque à l'appareil d'acquisition (à la différence de sa position et de son orientation) dont la valeur influence l'image acquise. De préférence, les paramètres de calibration sont choisis dans le groupe formé par l'ouverture de diaphragme, le temps d'exposition, la distance focale et la sensibilité.

Un scanner 3D est un appareil permettant d'obtenir une représentation en trois dimensions d'un objet.

Par "image", on entend une image en deux dimensions, comme une photographie. Une image est formée de pixels.

Une image actualisée « correspond à » une vue du modèle de référence actualisé lorsque cette vue est sensiblement identique à ladite image actualisée.

« Comprendre », « comporter » ou « présenter » doivent être interprétés de manière large, non limitative, sauf indication contraire.

### Brève description des figures

D'autres caractéristiques et avantages de l'invention apparaîtront encore à la lecture de la description détaillée qui va suivre et à l'examen du dessin annexé dans lequel :
- la figure 1 représente différentes évolutions temporelles pour un paramètre de positionnement d'une dent, en fonction de la nature du traitement résiduel considéré ;
- la figure 2 représente un logigramme illustrant la mise en oeuvre d'un procédé de prédiction selon l'invention.

### Description détaillée

**L'opération 1)** est de préférence réalisée en début de traitement ou avant le début du traitement, et consiste en la réalisation d'un modèle tridimensionnel numérique d'une arcade portant les dents traitées, ou « modèle de référence initial ».

Le modèle de référence initial est par exemple du type .stl ou .Obj, .DXF 3D, IGES, STEP, VDA, ou Nuages de points. Avantageusement, un tel modèle, dit « 3D », peut être observé selon un angle quelconque.

Le modèle de référence initial peut être préparé à partir de mesures effectuées sur les dents du patient ou sur un modèle physique de ses dents, par exemple un modèle en plâtre.

Le modèle de référence initial est de préférence créé au moyen d'un appareil professionnel, par exemple au moyen d'un scanner 3D, de préférence mis en oeuvre par un professionnel de la santé, par exemple par un orthodontiste ou un laboratoire d'orthodontie. Dans un cabinet d'orthodontie, le patient ou le modèle physique de ses dents peuvent être avantageusement disposés dans une position précise et l'appareil professionnel peut être perfectionné. Il en résulte un modèle de référence initial très précis. Le modèle de référence initial fournit de préférence une information sur le positionnement des dents avec une erreur inférieure à 5/10 mm, de préférence inférieure à 3/10 mm, de préférence inférieure à 1/10 mm.

Dans le modèle de référence initial, une partie qui correspond à une dent, ou « modèle de dent », est délimitée par un bord gingival qui peut être décomposé en un bord gingival intérieur (du côté de l'intérieur de la bouche par rapport à la dent), un bord gingival extérieur (orienté vers l'extérieur de la bouche par rapport à la dent) et deux bords gingivaux latéraux. Les modèles de dents peuvent être définis comme décrit, par exemple, dans la demande internationale PCT/EP2015/074896.

De préférence, le modèle de référence initial modélise également la gencive qui enserre les dents. La partie du modèle de référence initial qui représente la gencive est appelée « modèle de gencive ».

**L'opération 2)** comprend une modification du modèle de référence initial, par déplacement des modèles de dents, jusqu'à obtenir un positionnement des dents souhaité, dit « positionnement objectif ». Le positionnement objectif peut être celui souhaité à la fin du traitement (« set-up final ») ou à une étape intermédiaire prédéterminée du traitement (« set-up intermédiaire »).

De préférence, l'opération 2) comporte également une déformation du modèle de gencive. Dans un mode de réalisation préféré, la déformation du modèle de gencive est, au moins en partie, le résultat d'une simulation obtenue à partir du déplacement des modèles de dent. Avantageusement, la recherche du modèle de référence actualisé en est accélérée.

De préférence, l'opération 2), postérieure à l'opération 1), est réalisée immédiatement après l'opération 1).

Les opérations 3) et 4), postérieures à l'opération 2), ont pour objectif d'actualiser le modèle de référence initial pour que les modèles de dent soient dans un positionnement identique à celui des dents réelles à l'instant actuel.

**A l'opération 3),** on prend, au moyen d'un appareil d'acquisition d'images, dans des conditions d'acquisition réelles, une image actualisée de la partie de l'arcade portant les dents à traiter.

L'appareil d'acquisition d'images est de préférence un téléphone mobile, un appareil photo dit « connecté », une montre dite « intelligente », ou « smartwatch », une tablette ou un ordinateur personnel, fixe ou portable, comportant un système d'acquisition d'images, comme une webcam ou un appareil photo.

L'acquisition est de préférence réalisée par le patient ou un proche du patient, mais peut être réalisée par toute autre personne, notamment un dentiste ou un orthodontiste, de préférence sans imposer un positionnement précis de l'appareil d'acquisition d'images par rapport aux dents.

De préférence, l'image actualisée est une photographie ou est extraite d'un film. Elle est de préférence en couleurs, de préférence en couleurs réelles.

De préférence, à l'opération 3), on procède suivant l'étape b) décrite dans PCT/EP2015/074896.

**L'opération 4),** postérieure à l'opération 3), consiste de préférence en un processus itératif selon lequel, à chaque itération, un ou plusieurs modèles de dents sont déplacés, puis des conditions d'observation optimales du modèle de référence initial ainsi modifié (dit « modèle de référence à tester ») sont déterminées, les conditions optimales d'observation étant définies comme les conditions permettant d'observer le modèle de référence à tester de manière que la vue dudit modèle soit la plus proche possible de l'image actualisée.

De préférence, à chaque itération, le modèle de gencive est également déformé afin que le modèle de référence initial modifié par le déplacement des modèles de dent et la déformation du modèle de gencive soit le plus compatible possible avec l'image actualisée. Dans un mode de réalisation, une première déformation du modèle de gencive est calculée en fonction du déplacement des modèles de dents. La première déformation peut être suffisante. Sinon, elle est complétée par une deuxième déformation, de préférence déterminée au moyen d'une méthode métaheuristique, de préférence évolutionniste, de préférence par recuit simulé.

Les étapes c) à e) décrites dans PCT/EP2015/074896 sont de préférence mises en oeuvre :
c) analyse de l'image actualisée et réalisation d'une carte actualisée relative à une information discriminante ;
d) optionnellement, détermination, pour l'image actualisée, de conditions d'acquisition virtuelles grossières approximant les conditions d'acquisition réelles de ladite image actualisée;
e) recherche, pour l'image actualisée, d'un modèle de référence actualisé correspondant au positionnement des dents lors de l'acquisition de l'image actualisée, la recherche étant de préférence effectuée au moyen d'une méthode métaheuristique, de préférence évolutionniste, de préférence par recuit simulé.

Toutes les caractéristiques des étapes c) à e) décrites dans PCT/EP2015/074896 sont applicables.

Suivant l'étape c), l'image actualisée est analysée de manière à réaliser une carte actualisée relative à au moins une information discriminante.

Une "information discriminante" est une information caractéristique qui peut être extraite d'une image (*"image feature"*), classiquement par un traitement informatique de cette image.

Une information discriminante peut présenter un nombre variable de valeurs. Par exemple, une information de contour peut être égale à 1 ou 0 selon qu'un pixel appartient ou non à un contour. Une information de brillance peut prendre un grand nombre de valeurs. Le traitement de l'image permet d'extraire et de quantifier l'information discriminante.

La carte actualisée représente une information discriminante dans le référentiel de l'image actualisée. L'information discriminante est de préférence choisie dans le groupe constitué par une information de contour, une information de couleur, une information de densité, une information de distance, une information de brillance, une information de saturation, une information sur les reflets et des combinaisons de ces informations.

A l'étape optionnelle d), on évalue, de manière grossière, les conditions d'acquisition réelles de l'image actualisée à l'opération 3), c'est-à-dire la position et l'orientation dans l'espace de l'appareil d'acquisition par rapport aux dents et sa calibration. L'étape d) permet avantageusement de limiter le nombre de tests sur des conditions d'acquisition virtuelles lors de l'étape e), et permet donc d'accélérer considérablement l'étape e).

On utilise de préférence une ou plusieurs règles heuristiques. Par exemple, de préférence, on exclut des conditions d'acquisition virtuelles susceptibles d'être testées à l'étape e), les conditions qui correspondent à une position de l'appareil d'acquisition d'images derrière les dents ou à une distance des dents supérieure à 1 m. Dans un mode de réalisation préféré, on utilise des marques de repérage représentées sur l'image actualisée pour déterminer une région de l'espace sensiblement conique délimitant des conditions d'acquisition virtuelles susceptibles d'être testées à l'étape e), ou "cône de test".

L'objectif de l'étape e) est de modifier le modèle de référence initial jusqu'à obtenir un modèle de référence actualisé qui corresponde à l'image actualisée, c'est-à-dire tel qu'on puisse observer les modèles de dents comme représenté sur l'image actualisée. Idéalement, le modèle de référence actualisé est donc un modèle de référence tridimensionnel numérique à partir duquel l'image actualisée aurait pu être prise si ce modèle avait été réel.

On teste donc une succession de modèles de référence « à tester », le choix d'un modèle de référence à tester dépendant de préférence du niveau de correspondance des modèles de référence « à tester » précédemment testés avec l'image actualisée. Ce choix est de préférence effectué en suivant une méthode d'optimisation connue, en particulier choisie parmi les méthodes d'optimisation métaheuristiques, de préférence évolutionnistes, en particulier dans les procédés de recuit simulé. Les méthodes d'optimisation décrites dans PCT/EP2015/074896 sont notamment utilisables.

De préférence, l'étape e) comporte les étapes suivantes :
e1) définition d'un modèle de référence à tester comme étant le modèle de référence initial puis,
e2) suivant les étapes suivantes, test de conditions d'acquisition virtuelles avec le modèle de référence à tester afin d'approximer finement lesdites conditions d'acquisition réelles ;
   e21) détermination de conditions d'acquisition virtuelles à tester;
   e22) réalisation d'une image de référence bidimensionnelle du modèle de référence à tester dans lesdites conditions d'acquisition virtuelles à tester ;
   e23) traitement de l'image de référence pour réaliser au moins une carte de référence représentant, au moins partiellement, ladite information discriminante ;
   e24) comparaison des cartes actualisée et de référence de manière à déterminer une valeur pour une première fonction d'évaluation, ladite valeur pour la première fonction d'évaluation dépendant des différences entre lesdites cartes actualisée et de référence et correspondant à une décision de poursuivre ou de stopper la recherche de conditions d'acquisition virtuelles approximant lesdites conditions d'acquisition réelles avec plus d'exactitude que lesdites conditions d'acquisition virtuelles à tester déterminées à la dernière occurrence de l'étape e21) ;
   e25) si ladite valeur pour la première fonction d'évaluation correspond à une décision de poursuivre ladite recherche, modification des conditions d'acquisition virtuelles à tester, puis reprise à l'étape e22) ;
e3) détermination d'une valeur pour une deuxième fonction d'évaluation, ladite valeur pour la deuxième fonction d'évaluation dépendant des différences entre les cartes actualisée et de référence dans les conditions d'acquisition virtuelles approximant au mieux lesdites conditions d'acquisition réelles et résultant de la dernière occurrence de l'étape e2), ladite valeur pour la deuxième fonction d'évaluation correspondant à une décision de poursuivre ou de stopper la recherche d'un modèle de référence approximant le positionnement des dents lors de l'acquisition de l'image actualisée avec plus d'exactitude que ledit modèle de référence à tester utilisé à la dernière occurrence de l'étape e2), et si ladite valeur pour la deuxième fonction d'évaluation correspond à une décision de poursuivre ladite recherche, modification du modèle de référence à tester par déplacement d'un ou plusieurs modèles de dents, puis reprise à l'étape e2).

Les étapes e1) à e3) sont décrites en détail dans PCT/EP2015/074896, ou WO2016066651.

Suivant les étapes c) à e), le modèle de référence actualisé est un modèle tridimensionnel résultant de modifications successives du modèle de référence initial, très précis. Avantageusement, il est ainsi lui-même très précis, bien qu'il ait été obtenu à partir de simples photographies prises sans précautions particulières.

Ces étapes permettent donc, à partir d'une ou plusieurs simples images des dents, prises sans pré-positionnement précis de l'appareil d'acquisition d'images par rapport aux dents, par exemple à partir d'une photographie prise par le patient, d'évaluer avec précision la position des dents à l'instant actuel. Cette évaluation peut en outre être effectuée à distance, à partir de simples photographies prises par un téléphone mobile, sans que le patient ait à se déplacer, chez l'orthodontiste en particulier.

L'opération 4) conduit à un modèle de référence actualisé représentant les dents avec une grande précision, dans leur position à l'instant actuel.

Selon un premier mode de réalisation, le procédé se poursuit par la succession des étapes 5') à 8') :
**A l'étape 5')**, on détermine un ensemble de scénarios de traitement résiduel, c'est-à-dire adapté pour atteindre un positionnement des dents sensiblement identique à celui des modèles de dent correspondant dans le modèle de référence objectif.

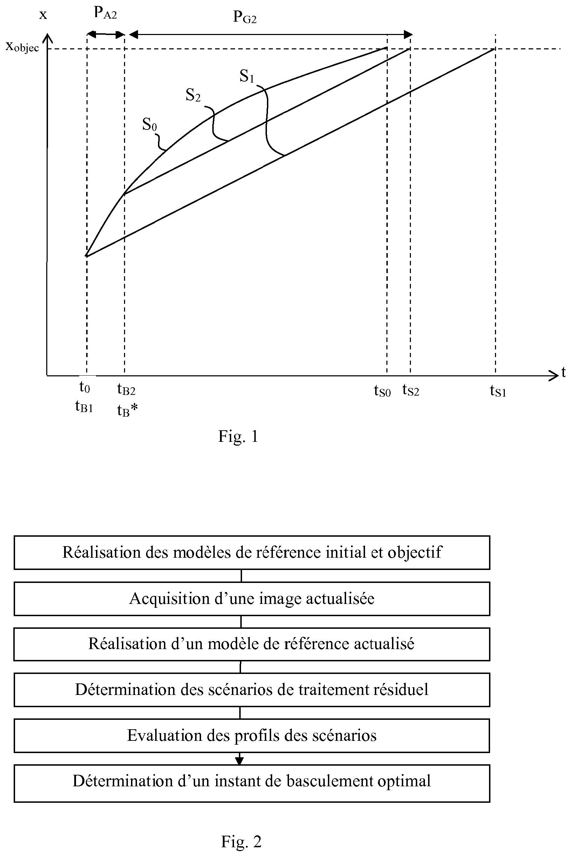

La figure 1 représente par exemple trois évolutions temporelles de la coordonnée x d'un point d'une dent dans l'espace, en fonction du traitement résiduel, c'est-à-dire du traitement adopté à partir de l'instant actuel t₀. Le positionnement objectif de ce point est atteint lorsque *x* est égal à *x_{objectif}.*

La courbe S₀ représente l'évolution de x si le traitement se poursuit exclusivement avec un appareil orthodontique à arc et attaches. Cette évolution est typique de ce type d'appareil orthodontique, avec une efficacité qui se réduit progressivement. Le traitement résiduel est le plus rapide, le positionnement objectif étant atteint à l'instant t_{S0}. La durée du port d'un appareil orthodontique à arc et attaches (t_{S0} - t₀) est cependant la plus longue.

La courbe S₁ représente un premier scénario pour un traitement mixte. Suivant ce scénario, l'appareil orthodontique à arc et attaches porté jusque-là est remplacé, à l'instant actuel, par une gouttière. L'instant de basculement t_{B1} est donc égal à t₀. Le traitement résiduel se poursuit alors exclusivement avec une ou plusieurs gouttières. L'évolution de x est dès lors sensiblement linéaire et illustre l'efficacité typique de ce type d'appareil orthodontique. La durée du port d'un appareil orthodontique à arc et attaches est avantageusement nulle. Le traitement résiduel cependant est le plus lent, le positionnement objectif étant atteint à l'instant t_{S1}.

La courbe S₂ représente un deuxième scénario pour un traitement mixte. Suivant ce scénario, l'appareil orthodontique à arc et attaches est porté, pendant une phase à arc P_{A2}, jusqu'à un instant de basculement t_{B2}, puis est remplacé par une gouttière. Le traitement résiduel se poursuit alors exclusivement avec une ou plusieurs gouttières (phase à gouttière P_{G2}).

La durée du port d'un appareil orthodontique à arc et attaches (t_{S2} - t_{B2}) et la durée du traitement résiduel sont intermédiaires entre celles des deux situations précédentes.

De préférence, plus de 3, plus de 5, plus de 10, plus de 50, ou plus de 100 scénarios de traitement résiduel mixte sont déterminés.

Les scénarios de traitement résiduel peuvent être déterminés par un orthodontiste et/ou au moyen de logiciels de simulation capables de simuler l'effet de différents appareils orthodontiques en fonction du positionnement actualisé des dents et du positionnement objectif, par exemple du logiciel Insignia^{™} de la société Ormco^{™}.

**A l'étape 6'),** on détermine un profil pour chaque scénario déterminé à l'étape 5'). Un « profil » est constitué d'un ensemble de valeurs pour des paramètres d'évaluation, c'est-à-dire pertinents pour évaluer un scénario en fonction d'une règle d'évaluation.

Les paramètres d'évaluation peuvent être relatifs à la durée, au coût, à la douleur, et/ou au confort, et/ou à la sévérité clinique associé à chaque scénario.

La douleur peut être mesurée au moyen d'un coefficient de douleur associé au traitement, par exemple évalué à partir de sondages effectués auprès de personnes ayant été traités de manière similaire.

Le confort peut en particulier faire référence à l'impact esthétique du traitement. Par exemple, le coefficient de confort peut être le rapport entre la durée totale des phases à gouttière sur la durée totale des phases à arc pour le traitement résiduel.

Le profil du scénario S₁ peut être par exemple (Durée de traitement résiduel = 3/10 ; Coefficient de confort = 9/10 ; Coefficient de douleur = 8/10 ; Coût = 7/10).

Le profil du scénario S₂ peut être par exemple (Durée de traitement résiduel = 7/10 ; Coefficient de confort = 6/10 ; Coefficient de douleur = 5/10 ; Coût = 9/10).

**A l'étape 7'),** les profils sont évalués au regard de la règle d'évaluation. La règle d'évaluation est de préférence établie par le patient, en fonction de l'importance qu'il attache aux paramètres d'évaluation. Elle peut être établie à tout moment avant l'étape 7'). De préférence, l'appareil d'acquisition pose des questions au patient et les réponses à ces questions lui permettent de définir la règle d'évaluation.

Le profil préféré, au regard de la règle d'évaluation, est dit « optimal ».

Une règle d'évaluation peut établir une contrainte. Par exemple, une règle d'évaluation peut imposer que la durée du traitement résiduel soit inférieure à 6 mois ou que l'ensemble des phases à arc du traitement complet dure moins de 2 mois.

Une règle d'évaluation peut établir une contrainte conditionnelle. Par exemple, une règle d'évaluation peut imposer que, si la durée du traitement résiduel est supérieure à 6 mois, l'ensemble des phases à arc du traitement résiduel ne doit pas durer plus de 2 mois.

Une règle d'évaluation peut établir une règle d'optimisation. Par exemple, une règle d'évaluation peut imposer de rechercher le traitement résiduel le plus court, avec une durée maximale de traitement avec un appareil orthodontique à arc et attaches de 1 mois.

Avec ce dernier exemple, le scénario S₂ pourrait être optimal si la durée entre t_{B2} et t₀ est de 1 mois.

Bien entendu, une règle d'évaluation peut être complexe et établir, par exemple, que l'ensemble des phases à arc du traitement résiduel dure le moins de temps possible, à un coût total déterminé.

Une règle d'évaluation pourrait être, dans l'exemple de la figure 1, de minimiser la fonction F = k₁^{∗}Durée de traitement résiduel + k₂^{∗}Coefficient de confort + k₃^{∗}Coefficient de douleur + k4^{∗}Coût, les coefficients de pondération kᵢ étant de préférence déterminés par le patient ou par un algorithme, en fonction de réponses du patient à des questions.

Si k₁=1, k₂=2, k₃=1 et k₄=3, la fonction F pour les scénarios S₁ et S₂ est égale, respectivement, à F(S₁) = 50 et F(S₂) = 51.

**A l'étape 8'),** on compare les évaluations des profils effectuées à l'étape 7') de manière à sélectionner un scénario considéré comme optimal au regard de la règle d'évaluation. L'instant de basculement dit « optimal » t_{B}^{∗} associé à ce scénario optimal peut alors être proposé, par exemple affiché sur un écran.

Dans l'exemple de la figure 1, F(S₁) (= 50) est inférieur à F(S₂) (= 51). Le profil de S₂ est donc optimal, S₂ est le scénario optimal et l'instant de basculement dit « optimal » t_{B}^{∗} est donc t_{B2}.

Dans un mode de réalisation préféré, les opérations 5) à 7) ne sont pas réalisées successivement. Les opérations 5) à 8) sont de préférence réalisées sous la forme des étapes 5") à 8").

Plus précisément, un premier scénario est déterminé et son profil évalué avant de déterminer un deuxième scénario. Le deuxième scénario peut ainsi, avantageusement, être déterminé en fonction de l'évaluation du premier scénario.

De préférence, le nouveau scénario de traitement résiduel est déterminé à partir des scénarios dont les profils ont été évalués précédemment. Par exemple, si les deux scénarios évalués précédemment montrent que l'augmentation de la durée des phases à arc dégrade le score des profils au regard de la règle d'évaluation, par exemple parce que cette règle est fortement influencée par un coefficient de confort, le nouveau scénario sera recherché parmi les scénarios présentant une durée totale des phases à arc réduite.

La recherche d'un scénario optimal est ainsi considérablement accélérée. Toutes les méthodes d'optimisation connues, peuvent être utilisées. La recherche du scénario optimal de préférence effectuée au moyen d'une méthode métaheuristique, de préférence évolutionniste, de préférence par recuit simulé, de préférence au moyen d'une méthode décrite dans PCT/EP2015/074896.

Les étapes 5") à 8") sont particulièrement bien adaptées lorsque la création de scénarios et l'évaluation des profils des scénarios est automatisée dans un simulateur. De préférence, un tel simulateur est programmé dans un outil de calcul, par exemple un ordinateur ou l'appareil d'acquisition, dans lequel la règle d'évaluation a été programmée.

Un simulateur de scénarios peut être créé à partir d'analyses statistiques de données historiques. En particulier, une analyse statistique de données historiques peut servir pour simuler l'effet d'un appareil orthodontique déterminé sur le positionnement de dents, mais aussi pour définir un profil, par exemple un coefficient de douleur ou un coefficient de confort pour le traitement résiduel en fonction de la durée des phases à arc, ou un coût de traitement complet en fonction des durées des phases à arc et à gouttière.

Dans un mode de réalisation préféré, le procédé comporte une étape 9) dans laquelle le scénario optimal et/ou le profil optimal sont présentés au patient, de préférence sur un écran, de préférence sur l'écran de son téléphone mobile. Bien entendu, le procédé peut conduire à la détermination de plusieurs scénarios optimaux. De préférence, tous les scénarios optimaux et/ou profils optimaux sont présentés au patient, de préférence sur l'appareil d'acquisition utilisé à l'étape 3).

Si le patient n'est pas satisfait, il peut établir une nouvelle règle d'évaluation, par exemple en modifiant les coefficients *kᵢ*, et reprendre le procédé à l'étape 5') ou 5").

L'instant de basculement optimal du scénario optimal retenu permet d'adapter précisément le traitement résiduel aux besoins du patient. En particulier, il est possible de fabriquer une ou plusieurs gouttières qui pourront être utilisées à partir de cet instant.

Comme cela apparaît clairement à présent, l'invention fournit un moyen efficace pour optimiser un traitement orthodontique mixte en fonction d'une règle d'évaluation. Avantageusement, cette règle d'évaluation peut prendre en compte de nombreux paramètres d'évaluation, ce qui permet de répondre précisément aux attentes spécifiques du patient. Enfin, un procédé selon l'invention peut être programmé, en particulier sur un téléphone mobile. A tout instant, le patient peut réaliser, à distance, des évaluations de différents scénarios et décider, en conséquence, de l'instant de basculement. Le procédé peut être mis en oeuvre autant de fois que souhaité, par exemple plus de 1 fois, plus de 2 fois, ou plus de 5 fois par mois.

Le nombre de rendez-vous chez l'orthodontiste est aussi avantageusement limité.

## Revendications

1. Procédé de détermination, par la mise en oeuvre d'un programme d'ordinateur, d'un instant de basculement optimal d'un traitement orthodontique mixte de dents d'un patient, ledit procédé comportant les opérations suivantes :
1) de préférence en début de traitement ou avant le début du traitement, réalisation d'un modèle tridimensionnel numérique d'au moins une partie d'une arcade portant lesdites dents, ou « modèle de référence initial », et pour chacune desdites dents, définition d'un modèle de référence tridimensionnel numérique de ladite dent dans le modèle de référence initial, ou « modèle de dent » ;
2) après l'opération 1), de préférence en début de traitement ou avant le début du traitement, déformation du modèle de référence initial, par déplacement des modèles de dent, jusqu'à ce que lesdits modèles de dents soient dans un positionnement objectif, de manière à obtenir un « modèle de référence objectif »,
le procédé étant **caractérisé par** les opérations suivantes :
3) après l'opération 2), à un instant d'une phase du traitement pendant laquelle le patient porte un appareil orthodontique à arc et attaches, dit « instant actuel », acquisition, au moyen d'un appareil d'acquisition d'images, d'au moins une image bidimensionnelle desdites dents, dite « image actualisée », dans des conditions d'acquisition réelles ;
4) déformation du modèle de référence initial, par déplacement des modèles de dent, jusqu'à ce que ladite au moins une image actualisée corresponde à une vue du modèle de référence initial ainsi déformé, dit « modèle de référence actualisé », la recherche du modèle de référence actualisé étant de préférence effectuée au moyen d'une méthode métaheuristique, de préférence évolutionniste, de préférence par recuit simulé ;
5) détermination, à partir dudit modèle de référence actualisé et dudit modèle de référence objectif, d'une pluralité de scénarios de traitement résiduel pour déplacer lesdites dents depuis leur position représentée dans le modèle de référence actualisé jusqu'à leur position représentée dans le modèle de référence objectif, chaque scénario comportant, à partir d'un instant de basculement, au moins une phase de traitement au moyen d'une gouttière orthodontique ;
6) détermination, pour chaque scénario, d'un profil comportant au moins une valeur pour un paramètre d'évaluation choisi dans le groupe formé par :
- la durée du traitement résiduel dudit scénario ;
- la durée de traitement complet ;
- un coefficient de complexité clinique ou « score de sévérité » ;
- un coefficient de douleur pour le traitement résiduel dudit scénario,
- un coefficient de douleur pour le traitement complet;
- la durée de la ou des phases de traitement au moyen d'un arc orthodontique à arc et attaches pendant le traitement résiduel ;
- un coefficient de confort pour le traitement résiduel dudit scénario,
- un coefficient de confort pour le traitement complet ;
- un coût pour le traitement résiduel dudit scénario,
- un coût pour le traitement complet,
- une fonction des paramètres d'évaluation précédents ;
7) évaluation de chaque profil au moyen d'une règle d'évaluation; et
8) détermination, à partir du scénario dont le profil est optimal, ou « scénario optimal », d'un instant de basculement optimal pour remplacer l'appareil orthodontique à arc et attaches par une gouttière.

2. Procédé selon la revendication 1, dans lequel plus de 50 scénarios sont déterminés à l'opération 5).

3. Procédé selon l'une quelconque des revendications précédentes, dans lequel les opérations 5) à 8) sont imbriquées de manière à se dérouler suivant les étapes 5") à 8") suivantes :
5") détermination, à partir dudit modèle de référence actualisé et dudit modèle de référence objectif, d'un scénario de traitement résiduel pour déplacer lesdites dents depuis leur position représentée dans le modèle de référence actualisé jusqu'à leur position dans le modèle de référence objectif, un scénario de traitement résiduel comportant, à partir d'un instant de basculement, au moins une phase de traitement au moyen d'une gouttière orthodontique, et, optionnellement, une ou plusieurs phase(s) de traitement au moyen d'un arc orthodontique à arc et attaches ;
6") détermination, pour ledit scénario, d'un profil comportant au moins une valeur pour un paramètre d'évaluation choisi dans le groupe formé par :
- la durée du traitement résiduel dudit scénario ;
- la durée de traitement complet ;
- un coefficient de complexité clinique ou « score de sévérité » ;
- un coefficient de douleur pour le traitement résiduel dudit scénario,
- un coefficient de douleur pour le traitement complet;
- la durée de la ou des phases de traitement au moyen d'un arc orthodontique à arc et attaches pendant le traitement résiduel ;
- un coefficient de confort pour le traitement résiduel dudit scénario,
- un coefficient de confort pour le traitement complet ;
- un coût pour le traitement résiduel dudit scénario,
- un coût pour le traitement complet,
- une fonction des paramètres d'évaluation précédents ;
7") évaluation du profil au moyen d'au moins une règle d'évaluation et, si le profil n'est pas optimal au regard de la règle d'évaluation, détermination d'un nouveau scénario de traitement résiduel et reprise à l'étape 6") ;
8") détermination, à partir du scénario dont le profil est optimal, ou « scénario optimal », d'un instant de basculement optimal pour remplacer l'appareil orthodontique à arc et attaches par une gouttière.

4. Procédé selon l'une quelconque des revendications précédentes, dans lequel, à l'opération 3), on prend l'image actualisée au moyen d'un appareil d'acquisition d'images choisi dans le groupe constitué par un téléphone mobile, un appareil photo connecté, une montre intelligente, une tablette et ou un ordinateur personnel, fixe ou portable.

5. Procédé selon l'une quelconque des revendications précédentes, dans lequel l'opération 4) comporte les étapes suivantes :
c) analyse de l'image actualisée et réalisation d'une carte actualisée relative à une information discriminante ;
d) optionnellement, détermination, pour l'image actualisée, de conditions d'acquisition virtuelles grossières approximant les conditions d'acquisition réelles de ladite image actualisée;
e) recherche, pour l'image actualisée, d'un modèle de référence actualisé correspondant au positionnement des dents lors de l'acquisition de l'image actualisée, la recherche étant de préférence effectuée au moyen d'une méthode métaheuristique, de préférence évolutionniste, de préférence par recuit simulé.

6. Procédé selon la revendication immédiatement précédente, dans lequel l'étape e) comporte les étapes suivantes :
e1) définition d'un modèle de référence à tester comme étant le modèle de référence initial puis,
e2) suivant les étapes suivantes, test de conditions d'acquisition virtuelles avec le modèle de référence à tester afin d'approximer finement lesdites conditions d'acquisition réelles ;
e21) détermination de conditions d'acquisition virtuelles à tester;
e22) réalisation d'une image de référence bidimensionnelle du modèle de référence à tester dans lesdites conditions d'acquisition virtuelles à tester ;
e23) traitement de l'image de référence pour réaliser au moins une carte de référence représentant, au moins partiellement, ladite information discriminante ;
e24) comparaison des cartes actualisée et de référence de manière à déterminer une valeur pour une première fonction d'évaluation, ladite valeur pour la première fonction d'évaluation dépendant des différences entre lesdites cartes actualisée et de référence et correspondant à une décision de poursuivre ou de stopper la recherche de conditions d'acquisition virtuelles approximant lesdites conditions d'acquisition réelles avec plus d'exactitude que lesdites conditions d'acquisition virtuelles à tester déterminées à la dernière occurrence de l'étape e21) ;
e25) si ladite valeur pour la première fonction d'évaluation correspond à une décision de poursuivre ladite recherche, modification des conditions d'acquisition virtuelles à tester, puis reprise à l'étape e22) ;
e3) détermination d'une valeur pour une deuxième fonction d'évaluation, ladite valeur pour la deuxième fonction d'évaluation dépendant des différences entre les cartes actualisée et de référence dans les conditions d'acquisition virtuelles approximant au mieux lesdites conditions d'acquisition réelles et résultant de la dernière occurrence de l'étape e2), ladite valeur pour la deuxième fonction d'évaluation correspondant à une décision de poursuivre ou de stopper la recherche d'un modèle de référence approximant le positionnement des dents lors de l'acquisition de l'image actualisée avec plus d'exactitude que ledit modèle de référence à tester utilisé à la dernière occurrence de l'étape e2), et si ladite valeur pour la deuxième fonction d'évaluation correspond à une décision de poursuivre ladite recherche, modification du modèle de référence à tester par déplacement d'un ou plusieurs modèles de dents, puis reprise à l'étape e2).

7. Procédé selon l'une quelconque des revendications précédentes, comportant une étape 9) dans laquelle l'instant de basculement optimal, de préférence le scénario optimal et/ou le profil optimal, sont présentés au patient.

8. Procédé selon l'une quelconque des revendications précédentes, dans lequel, à l'étape 1), on définit un modèle de référence tridimensionnel numérique d'une gencive de laquelle émergent lesdits modèles de dents, ou « modèle de gencive », et, à l'étape 4), on déplace lesdits modèles de dent et on modifie ledit modèle de gencive pour déformer le modèle de référence initial.

9. Procédé selon la revendication immédiatement précédente, dans lequel pour déformer le modèle de référence initial, on calcule une déformation du modèle de gencive à partir desdits déplacements desdits modèles de dent.

10. Outil de calcul, de préférence téléphone mobile, comportant un programme comprenant des instructions de code de programme pour l'exécution des opérations 3) à 8) d'un procédé selon l'une quelconque des revendications précédentes, lorsque ledit programme est exécuté.

11. Outil de calcul selon la revendication immédiatement précédente, comportant un simulateur de scénarios apte à créer des scénarios, à déterminer des profils desdits scénarios et à évaluer lesdits scénarios au regard d'une règle d'évaluation.

12. Outil de calcul selon la revendication immédiatement précédente, comportant une interface « homme-machine » et des moyens pour déterminer la règle d'évaluation à partir de données saisies avec ladite interface.

## Patentansprüche

1. Verfahren zur Bestimmung, durch die Anwendung eines Computerprogramms, eines optimalen Umstellungszeitpunkts einer gemischten kieferorthopädischen Behandlung von Zähnen eines Patienten, wobei das Verfahren die folgenden Vorgänge aufweist:
1) vorzugsweise zu Beginn einer Behandlung oder vor dem Beginn der Behandlung, Herstellung eines digitalen dreidimensionalen Modells mindestens eines Teils eines die Zähne tragenden Zahnbogens, oder "Ausgangsbezugsmodell", und für jeden der Zähne Definition eines digitalen dreidimensionalen Bezugsmodells des Zahns in einem Ausgangsbezugsmodell, oder "Zahnmodell";
2) nach dem Arbeitsgang 1), vorzugsweise zu Beginn der Behandlung oder vor dem Beginn der Behandlung, Verformung des Ausgangsbezugsmodells durch Verschiebung der Zahnmodelle, bis die Zahnmodelle sich in einer objektiven Positionierung befinden, um ein "objektives Bezugsmodell" zu erhalten,
wobei das Verfahren durch die folgenden Vorgänge gekennzeichnet ist:
3) nach dem Arbeitsgang 2), zu einem Zeitpunkt einer Phase der Behandlung, während der der Patient eine kieferorthopädische Apparatur mit Bogen und Brackets trägt, "aktueller Zeitpunkt" genannt, Erfassung, mittels eines Bilderfassungsgeräts, mindestens eines zweidimensionalen Bilds der Zähne, "aktualisiertes Bild" genannt, unter realen Erfassungsbedingungen;
4) Verformung des Ausgangsbezugsmodells durch Verschiebung der Zahnmodelle, bis das mindestens eine aktualisierte Bild einer Ansicht des so verformten Ausgangsbezugsmodells entspricht, "aktualisiertes Bezugsmodell" genannt, wobei die Suche des aktualisierten Bezugsmodells vorzugsweise mittels einer metaheuristischen, vorzugsweise evolutionären Methode, vorzugsweise durch simuliertes Annealing ausgeführt wird;
5) Bestimmung, ausgehend vom aktualisierten Bezugsmodell und vom objektiven Bezugsmodell, einer Vielzahl von Restbehandlungsszenarien, um die Zähne von ihrer im aktualisierten Bezugsmodell dargestellten Position bis in ihre im objektiven Bezugsmodell dargestellte Position zu verschieben, wobei jedes Szenario ausgehend von einem Umstellungszeitpunkt mindestens eine Behandlungsphase mittels einer Zahnschiene aufweist;
6) Bestimmung, für jedes Szenario, eines Profils, das mindestens einen Wert für einen Bewertungsparameter aufweist, der aus der Gruppe ausgewählt wird, die gebildet wird von:
- der Dauer der Restbehandlung des Szenarios;
- der vollständigen Behandlungsdauer;
- einem Koeffizienten klinischer Komplexität oder "Schweregrad-Score";
- einem Schmerzkoeffizienten für die Restbehandlung des Szenarios,
- einem Schmerzkoeffizienten für die vollständige Behandlung;
- der Dauer der Behandlungsphase(n) mittels eines kieferorthopädischen Bogens mit Bogen und Brackets während der Restbehandlung;
- einem Komfortkoeffizienten für die Restbehandlung des Szenarios,
- einem Komfortkoeffizienten für die vollständige Behandlung;
- Kosten für die Restbehandlung des Szenarios,
- Kosten für die vollständige Behandlung,
- einer Funktion der vorhergehenden Bewertungsparameter;
7) Bewertung jedes Profils mittels einer Bewertungsregel; und
8) Bestimmung, ausgehend von dem Szenario, dessen Profil optimal ist, oder "optimalen Szenario", eines optimalen Umstellungszeitpunkts, um die kieferorthopädische Apparatur mit Bogen und Brackets durch eine Schiene zu ersetzen.

2. Verfahren nach Anspruch 1, wobei mehr als 50 Szenarien im Arbeitsgang 5) bestimmt werden.

3. Verfahren nach einem der vorhergehenden Ansprüche, wobei die Vorgänge 5) bis 8) so verschachtelt sind, dass sie gemäß den folgenden Schritten 5") bis 8") ablaufen:
5") Bestimmung, ausgehend vom aktualisierten Bezugsmodell und vom objektiven Bezugsmodell, eines Restbehandlungsszenarios zur Verschiebung der Zähne von ihrer im Bezugsmodell dargestellten Position in ihre Position im objektiven Bezugsmodell, wobei ein Restbehandlungsszenario ausgehend von einem Umstellungszeitpunkt mindestens eine Behandlungsphase mittels einer kieferorthopädischen Schiene und optional eine oder mehrere Behandlungsphase(n) mittels eines kieferorthopädischen Bogens mit Bogen und Brackets aufweist;
6") Bestimmung, für das Szenario, eines Profils, das mindestens einen Wert für einen Bewertungsparameter aufweist, der aus der Gruppe ausgewählt wird, die gebildet wird von:
- der Dauer der Restbehandlung des Szenarios;
- der vollständigen Behandlungsdauer;
- einem Koeffizienten klinischer Komplexität oder "Schweregrad-Score";
- einem Schmerzkoeffizienten für die Restbehandlung des Szenarios,
- einem Schmerzkoeffizienten für die vollständige Behandlung;
- der Dauer der Behandlungsphase(n) mittels eines kieferorthopädischen Bogens mit Bogen und Brackets während der Restbehandlung;
- einem Komfortkoeffizienten für die Restbehandlung des Szenarios,
- einem Komfortkoeffizienten für die vollständige Behandlung;
- Kosten für die Restbehandlung des Szenarios,
- Kosten für die vollständige Behandlung,
- einer Funktion der vorhergehenden Bewertungsparameter;
7") Bewertung des Profils mittels mindestens einer Bewertungsregel, und wenn das Profil im Hinblick auf die Bewertungsregel nicht optimal ist, Bestimmung eines neuen Restbehandlungsszenarios und Wiederbeginn im Schritt 6");
8") Bestimmung, ausgehend von dem Szenario, dessen Profil optimal ist, oder "optimalen Szenario", eines optimalen Umstellungszeitpunkts, um die kieferorthopädische Apparatur mit Bogen und Brackets durch eine Schiene zu ersetzen.

4. Verfahren nach einem der vorhergehenden Ansprüche, wobei im Arbeitsgang 3) das aktualisierte Bild mittels eines Bilderfassungsgeräts aufgenommen wird, das aus der Gruppe ausgewählt wird, die aus einem Mobiltelefon, einem verbundenen Fotoapparat, einer intelligenten Uhr, einem Tablet und/oder einem ortsfesten oder tragbaren persönlichen Computer besteht.

5. Verfahren nach einem der vorhergehenden Ansprüche, wobei der Arbeitsgang 4) die folgenden Schritte aufweist:
c) Analyse des aktualisierten Bilds und Herstellung einer aktualisierten Karte bezüglich einer Diskriminanzinformation;
d) optional Bestimmung für das aktualisierte Bild von groben virtuellen Erfassungsbedingungen, die sich den realen Erfassungsbedingungen des aktualisierten Bilds annähern;
e) Suche, für das aktualisierte Bild, eines aktualisierten Bezugsmodells entsprechend der Positionierung der Zähne bei der Erfassung des aktualisierten Bilds, wobei die Suche vorzugsweise mittels einer metaheuristischen, vorzugsweise evolutionären Methode ausgeführt wird, vorzugsweise durch simuliertes Annealing.

6. Verfahren nach dem direkt vorhergehenden Anspruch, wobei der Schritt e) die folgenden Schritte aufweist:
e1) Definition eines zu testenden Bezugsmodells als das Ausgangsbezugsmodell, dann
e2) gemäß den folgenden Schritten, Test von virtuellen Erfassungsbedingungen mit dem zu testenden Bezugsmodell, um sich den realen Erfassungsbedingungen fein anzunähern;
e21) Bestimmung zu testender virtueller Erfassungsbedingungen;
e22) Herstellung eines zweidimensionalen Bezugsbilds des zu testenden Bezugsmodells unter den zu testenden virtuellen Erfassungsbedingungen;
e23) Verarbeitung des Bezugsbilds, um mindestens eine Bezugskarte herzustellen, die zumindest teilweise die Diskriminanzinformation darstellt;
e24) Vergleich der aktualisierten und der Bezugskarte, um einen Wert für eine erste Bewertungsfunktion zu bestimmen, wobei der Wert für die erste Bewertungsfunktion von den Differenzen zwischen der aktualisierten und der Bezugskarte abhängt und einer Entscheidung für das Fortsetzen oder Beenden der Suche von virtuellen Erfassungsbedingungen entspricht, die sich den realen Erfassungsbedingungen mit größerer Genauigkeit annähern als die zu testenden virtuellen Erfassungsbedingungen, die beim letzten Vorkommen des Schritts e21) bestimmt wurden;
e25) wenn der Wert für die erste Bewertungsfunktion einer Entscheidung der Fortsetzung der Suche entspricht, Änderung der zu testenden virtuellen Erfassungsbedingungen, dann Wiederbeginn im Schritt e22);
e3) Bestimmung eines Werts für eine zweite Bewertungsfunktion, wobei der Wert für die zweite Bewertungsfunktion von den Differenzen zwischen der aktualisierten und der Bezugskarte unter den virtuellen Erfassungsbedingungen abhängt, die sich am besten den realen Erfassungsbedingungen annähern und aus dem letzten Vorkommen des Schritts e2) resultieren, wobei der Wert für die zweite Bewertungsfunktion einer Entscheidung entspricht, die Suche eines Bezugsmodells, das sich der Positionierung der Zähne bei der Erfassung des aktualisierten Bilds mit größerer Genauigkeit annähert als das zu testende Bezugsmodell, das beim letzten Vorkommen des Schritts e2) verwendet wurde, fortzusetzen oder zu beenden, und wenn der Wert für die zweite Bewertungsfunktion einer Entscheidung für die Fortsetzung der Suche entspricht, Änderung des zu testenden Bezugsmodells durch Verschieben eines oder mehrerer Zahnmodelle, dann Wiederbeginn im Schritt e2).

7. Verfahren nach einem der vorhergehenden Ansprüche, das einen Schritt 9) aufweist, in dem der optimale Umstellungszeitpunkt, vorzugsweise das optimale Szenario und/oder das optimale Profil, dem Patienten präsentiert wird.

8. Verfahren nach einem der vorhergehenden Ansprüche, wobei im Schritt 1) ein digitales dreidimensionales Bezugsmodell eines Zahnfleischs definiert wird, aus dem die Zahnmodelle austreten, oder "Zahnfleischmodell", und im Schritt 4) die Zahnmodelle verschoben werden und das Zahnfleischmodell geändert wird, um das Ausgangsbezugsmodell zu verformen.

9. Verfahren nach dem direkt vorhergehenden Anspruch, wobei zum Verformen des Ausgangsbezugsmodells eine Verformung des Zahnfleischmodells ausgehend von den Verschiebungen der Zahnmodelle berechnet wird.

10. Rechenwerkzeug, vorzugsweise Mobiltelefon, das ein Programm aufweist, das Programmcodeanweisungen zur Ausführung der Arbeitsgänge 3) bis 8) eines Verfahrens nach einem der vorhergehenden Ansprüche enthält, wenn das Programm ausgeführt wird.

11. Rechenwerkzeug nach dem direkt vorhergehenden Anspruch, das einen Szenarien-Simulator aufweist, der fähig ist, Szenarien zu erzeugen, Profile der Szenarien zu bestimmen und die Szenarien im Hinblick auf eine Bewertungsregel zu bewerten.

12. Rechenwerkzeug nach dem direkt vorhergehenden Anspruch, das eine "Mensch-Maschine"-Schnittstelle und Einrichtungen aufweist, um die Bewertungsregel ausgehend von mit der Schnittstelle eingegebenen Daten zu bestimmen.

## Claims

1. Method for determining, by the implementation of a computer program, an optimal instant of switchover of a mixed orthodontic treatment of teeth of a patient, said method comprising the following operations:
1) preferably at the start of treatment or before the start of the treatment, production of a digital three-dimensional model of at least a part of an arch bearing said teeth, or "initial reference model", and, for each of said teeth, definition of a digital three-dimensional reference model of said tooth in the initial reference model, or "tooth model";
2) after the operation 1), preferably at the start of treatment or before the start of the treatment, deformation of the initial reference model, by displacement of the tooth models, until said tooth models are in an objective positioning, so as to obtain an "objective reference model",
the method being **characterized by** the following operations:
3) after the operation 2), at an instant of a phase of the treatment during which the patient wears an orthodontic appliance with arch and attachments, called "current instant", acquisition, by means of an image acquisition device, of at least one two-dimensional image of said teeth, called "updated image", in real acquisition conditions;
4) deformation of the initial reference model, by displacement of the tooth models, until said at least one updated image corresponds to a view of the initial reference model thus deformed, called "updated reference model", the search for the updated reference model being preferably performed by means of a metaheuristic method, preferably evolutionist, preferably by simulated annealing;
5) determination, from said updated reference model and from said objective reference model, of a plurality of remaining treatment scenarios for displacing said teeth from their position represented in the updated reference model to their position represented in the objective reference model, each scenario comprising, from an instant of switchover, at least one treatment phase by means of an orthodontic aligner;
6) determination, for each scenario, of a profile comprising at least one value for an evaluation parameter chosen from the group formed by:
- the duration of the remaining treatment of said scenario;
- the complete treatment duration;
- a clinical complexity coefficient or "severity score";
- a pain coefficient for the remaining treatment of said scenario,
- a pain coefficient for the complete treatment;
- the duration of the treatment phase or phases by means of an orthodontic arch with arch and attachments during the remaining treatment;
- a comfort coefficient for the remaining treatment of said scenario,
- a comfort coefficient for the complete treatment;
- a cost for the remaining treatment of said scenario,
- a cost for the complete treatment,
- a function of the above evaluation parameters;
7) evaluation of each profile by means of an evaluation rule; and
8) determination, from the scenario for which the profile is optimal, or "optimal scenario", of an optimal instant of switchover to replace the orthodontic appliance with arch and attachments with an aligner.

2. Method according to Claim 1, wherein more than 50 scenarios are determined in the operation 5).

3. Method according to either one of the preceding claims, wherein the operations 5) to 8) are interleaved so as to proceed according to the following steps 5") to 8"):
5") determination, from said updated reference model and from said objective reference model, of a remaining treatment scenario for displacing said teeth from their position represented in the updated reference model to their position in the objective reference model, a remaining treatment scenario comprising, from an instant of switchover, at least one treatment phase by means of an orthodontic aligner, and, optionally, one or more treatment phases by means of an orthodontic arch with arch and attachments;
6") determination, for said scenario, of a profile comprising at least one value for an evaluation parameter chosen from the group formed by:
- the duration of the remaining treatment of said scenario;
- the complete treatment duration;
- a clinical complexity coefficient or "severity score";
- a pain coefficient for the remaining treatment of said scenario,
- a pain coefficient for the complete treatment;
- the duration of the treatment phase or phases by means of an orthodontic arch with arch and attachments during the remaining treatment;
- a comfort coefficient for the remaining treatment of said scenario,
- a comfort coefficient for the complete treatment;
- a cost for the remaining treatment of said scenario,
- a cost for the complete treatment,
- a function of the above evaluation parameters;
7") evaluation of the profile by means of at least one evaluation rule and, if the profile is not optimal in light of the evaluation rule, determination of a new remaining treatment scenario and resumption at the step 6");
8") determination, from the scenario for which the profile is optimal, or "optimal scenario", of an optimal instant of switchover to replace the orthodontic appliance with arch and attachments with an aligner.

4. Method according to any one of the preceding claims, wherein, in the operation 3), the updated image is taken by means of an image acquisition device chosen from the group composed of a mobile phone, a connected camera, a smart watch, a tablet and/or a personal computer, fixed or portable.

5. Method according to any one of the preceding claims, wherein the operation 4) comprises the following steps:
c) analysis of the updated image and production of an updated map relating to a discriminating piece of information;
d) optionally, determination, for the updated image, of rough virtual acquisition conditions approximating the real acquisition conditions of said updated image;
e) search, for the updated image, for an updated reference model corresponding to the positioning of the teeth upon the acquisition of the updated image, the search being preferably performed by means of a metaheuristic method, preferably evolutionist, preferably by simulated annealing.

6. Method according to the immediately preceding claim, wherein the step e) comprises the following steps:
e1) definition of a reference model to be tested as being the initial reference model then,
e2) following the following steps, testing of virtual acquisition conditions with the reference model to be tested in order to finely approximate said real acquisition conditions;
e21) determination of virtual acquisition conditions to be tested;
e22) production of a two-dimensional reference image of the reference model to be tested in said virtual acquisition conditions to be tested;
e23) processing of the reference image to produce at least one reference map representing, at least partially, said discriminating piece of information;
e24) comparison of the updated and reference maps so as to determine a value for a first evaluation function, said value for the first evaluation function depending on the differences between said updated and reference maps and corresponding to a decision to continue or to stop the search for virtual acquisition conditions approximating said real acquisition conditions with greater precision than said virtual acquisition conditions to be tested determined on the last occurrence of the step e21);
e25) if said value for the first evaluation function corresponds to a decision to continue said search, modification of the virtual acquisition conditions to be tested, then resumption at the step e22);
e3) determination of a value for a second evaluation function, said value for the second evaluation function depending on the differences between the updated and reference maps in the virtual acquisition conditions best approximating said real acquisition conditions and resulting from the last occurrence of the step e2), said value for the second evaluation function corresponding to a decision to continue or to stop the search for a reference model approximating the positioning of the teeth upon the acquisition of the updated image with greater precision than said reference model to be tested used on the last occurrence of the step e2) and, if said value for the second evaluation function corresponds to a decision to continue said search, modification of the reference model to be tested by displacement of one or more tooth models, then resumption at the step e2).

7. Method according to any one of the preceding claims, comprising a step 9) in which the optimal instant of switchover, preferably the optimal scenario and/or the optimal profile, are presented to the patient.

8. Method according to any one of the preceding claims, wherein, in the step 1), a digital three-dimensional reference model of a gingiva from which said tooth models emerge, or "gingiva model", is defined, and, in the step 4), said tooth models are displaced and said gingiva model is modified to deform the initial reference model.

9. Method according to the immediately preceding claim, wherein, to deform the initial reference model, a deformation of the gingiva model is calculated on the basis of said displacements of said tooth models.

10. Computation tool, preferably a mobile phone, comprising a program comprising program code instructions for the execution of the operations 3) to 8) of a method according to any one of the preceding claims, when said program is run.

11. Computation tool according to the immediately preceding claim, comprising a scenario simulator capable of creating scenarios, of determining profiles of said scenarios and of evaluating said scenarios in light of an evaluation rule.

12. Computation tool according to the immediately preceding claim, comprising a "human-machine" interface and means for determining the evaluation rule on the basis of data input with said interface.
